# EUROPEAN PATENT SPECIFICATION

(11) **EP 0 286 163 B1**
(45) Date of publication and mention of the grant of the patent: **03.03.1993**
(21) Application number: 88200568.9
(22) Date of filing: 25.03.1988
(51) Int. Cl.: A61L 33/00, B32B 1/08, B32B 27/08, A61L 29/00

(54) **Tubular apparatuses of plastics, for bio-medical use**
Rohrförmige Gegenstände aus Kunststoff für biomedizinische Anwendung
Objets tubulaires en plastique pour usage biomédical

(30) Priority: 08.04.1987 IT 2002287
(43) Date of publication of application: 12.10.1988
(73) Proprietor: BELLCO S.p.A., I-41037 Mirandola(Modena) (IT)
(72) Inventor: CASALI, Enzo, IT-27100 PAVIA (IT)
(74) Representative: Boggio, Luigi

(56) References cited:
- EP-A- 0 232 171
- US-A- 4 627 844

## Description

The present invention relates to tubular elements advantageously useable in the bio-medical field, which are constituted by a double-layer plastics material, formed by an inner layer of poly(ethylene-co-vinyl acetate) and an outer layer of poly(vinyl chloride), wherein the inner layer has a thickness comprised within the range of from 35 to 95% of the total thickness of the tubular structure.

At present, a wide range of medical-surgical devices exist, which are used in the techniques of extra-corporeal blood circulation, such as, e.g., in haemodialysis or in cardiosurgery, or which are more generally destined to come into contact with human blood, such as, e.g., in perfusion, transfusion or blood - or its fractions - drawing systems.

Such devices are mostly constituted by connection tubular apparatuses having different size and different mechanical characteristics according to the particular type of expected use, and by a minor portion of fittings and components of various utility.

In general, the plastics material used for preparing these tubular elements is poly(vinyl chloride), briefly indicated as PVC.

PVC in fact, besides having a high chemical stability, has a unique versatility, which makes it possible, according to the type and amount of the additives it is compounded with, an extremely wide range of materials having extremely different chemical, physical and biological characteristics, to be obtained. The present use of PVC materials ranges hence from materials for building industry, to insulating products, to the disposables in bio-medical field, and so forth.

PVC is furthermore endowed with the advantage that it is a thermoplastic polymer, and that it can hence be extruded into the desired shapes.

PVC is never used alone, but is always blended with other ingredients before being processed. In particular, when obtaining a flexible, plastic, more or less soft material is desired, such as, e.g., in case of the tubular structures for bio-medical uses, it is necessary to resort to large amounts of additives in polymer compounding. Generally, besides minor amounts of stabilizer and lubricant additives, PVC shall also contain an amount larger than 25%, and preferably larger than 30%, of a plastifier.

Stabilizers conventionally used in PVC compounding are based on metal salts of either inorganic or organic acids, phenolic compounds, organometallic compounds, epoxy compounds, and phosphates, often used in combination with one another. As relates to the bio-medical field, the additives approved by the relevant control entities, such as the Ministry of Health in Italy, FDA in the United States, and so forth, are blends of organic salts of calcium, magnesium and zinc, and of epoxydated compounds, such as vegetable oils, e.g., epoxydated soybean oil and linseed-oil, or epoxydated esters of fatty acids, e.g., the octyl ester, or the decyl ester of epoxy-stearic acid. Whilst the metal salts are normally used in percent amounts smaller than, or equal to, 1%, the epoxydated compounds are used in amounts generally comprised within the range of from 5 to 10%.

Lubricating additives, generally esters of fatty acids, and, in particular, stearates, are used in very minor amounts, typically smaller than 1%.

The largest additive percentage, as above pointed out, is precisely constituted by the plastifiers. The plastifiers useable in the preparation of flexible PVC for general use, as well as the methods for compounding PVC with the suitable additives, are well known from technical literature (see, e.g., Kirk-Othmer, Encyclopaedia of Chemical Technology, 3^{rd} Edition (1983), Vol. 18, pages 133-152).

On considering that such large amounts of plastifier relatively to the polymer are used, and that the plastifier molecule is not permanently bound to the resin, and can hence be extracted to a larger or smaller extent by the liquid which is contained inside the PVC vessel, or which flows through the PVC pipe, it is evident how this is the aspect to be better controlled when PVC for use in medical-surgical field is compounded.

In particular, the plastifiers approved for bio-medical uses, e.g., by FDA, belong to the class consisting of the di- or tri-esters of organic acids, and, in particular, to the class of phthalates and adipates, such as, e.g., di(2-ethylhexyl) phthalate (DOP), diethyl phthalate, benzyl butyl phthalate (BBP), dicyclohexyl phthalate, di(2-ethylhexyl) adipate, tributyl citrate, glyceryl triacetate, and so forth. Among these, the most widely and advantageously used plastifier is DOP. The Italian Pharmacopoeia prescribes that the containers for human blood on the basis of plastified PVC should not contain more than 40% of DOP. It furthermore, as regards the requisites the apparatuses for haemodialisys, wherein the flexible connections are generally made with plastified PVC pipes, should meet, prescribes a maximum concentration limit, as ppm, for phthalates extracted by blood from the plastics material. Also the methodology of the test for phthalate determination is reported. Such determination is carried out by circulating through the apparatus whole milk, a substance which contains a lipophylic fraction very similar to the plasmatic lipophylic fraction.

In fact, phthalates, which seems to us to have been demonstrated to have a mutagen activity, do not show a high extraction resistance at such high concentrations as used for preparing the tubular structures.

Attempts to reduce as much as possible the amount of plastifier which migrates or diffuses from the plastics material to the blood contained inside it, resort, e.g., to the use of a gel hydrate, obtained from gelatin or collagen, as the inner lining for the PVC pipe (see Japanese patent application publ. No. 46961/83 - Derwent Card 40509k) or the use of a thin inner layer of silicone rubber (see EP-A-99220).

Besides the technical and economic problems typical for the production of these inner linings, in that in both cases a step of preparation or distribution of the linings is envisaged, which is separate from the PVC pipe production step, the obtainment, in the first case, of a gel hydrate, and, in the second case, of a thin layer of silicone rubber, does not completely solve the problem of the migration of the plastifiers and of the antioxidants, in that the total amount of these additives, and, in particular, of the plastifier, remains large, and the thin coating does not represent a barrier against its diffusion. Furthermore, as regards in particular the equipment for haemodialysis, wherein a portion of the tubular structure should show a considerable mechanical strength, in that it is subject to the action of the peristaltic pump, the mechanical strength of such a coating is definitely very low.

The present Applicant has found now that it is possible to obviate the problems caused by the migration of the additives, and, in particular, of the plastifiers, with at the same time the advantages being retained, in terms of versatility, processing easiness and low cost, which are typical for PVC, if for medical-surgical use double-layer tubular elements of plastics material are used, which are characterized in that with an outer layer constituted by PVC, an inner layer - which comes into contact with blood or with its fractions - is coupled, which is constituted by poly(ethylene-co-vinyl acetate), more briefly indicated as EVA, and that the thickness of the inner EVA layer is comprised within the range of from 35 to 95% of the total thickness of the tubular structure.

According to a preferred aspect of the invention, the thickness of the inner layer will be comprised within the range of from 70 to 85%, in that thicknesses within this range will better balance the reduction in phthalate contents in the eluate with the easiness of assemblage of the tubular structures.

The preparation of these tubular structures is very easily carried out by using the well-known co-extrusion technique (see Mark-Bikales et al., Encyclopaedia of Polymer Sciences and Engineering, 2^{nd} Ed., Vol. 6, pages 608-foll, and therein cited references).

The poly(ethylene-co-vinyl acetate) (EVA) which can be used in the co-extrusion process according to the invention for preparing the double-layer tubular structures, will generally contain from 6.5 to 40% of vinyl acetate according to the desired mechanical characteristics, typically of flexibility and elasticity. However, for preparing tubular structures for use in bio-medical field, such a percentage shall preferably be comprised within the range of from 15 to 25%. However, no limitations exist in use between the different types of EVA and the different types of PVC.

The co-extrusion procedure is carried out by separately heating both thermoplastic materials to their respective melting temperatures, or, in case of EVA, which has a higher temperature resistance, also to temperatures higher than melting temperature, simultaneously extruding these two materials, substantially at these temperatures, through a suitable co-extrusion head, and then cooling the so-extruded tubular structure, e.g., by passing it, under a slight pull, through a water cooling bath. In this particular case, in as much as the thermoplastic material which constitutes the inner layer of the tubular has a hardening temperature lower than that of the external material, in order to prevent the inner layer from collapsing when exiting the co-extrusion head, a very low air overpressure (of the order of a few mm_{Hg}) is maintained inside the tube, inside its length running from the extrusion head to the water cooling bath.

The so-obtained tubular structure can be then assembled according to the conventional techniques as used for tubular structures totally consisting of PVC. In particular, the glueing technique may be advantageously used, by using cyclohexanone or other suitable solvents for stably binding the tubular structure to the various components or fittings of the particular apparatus.

Both the composition of each thermoplastic material used in the preparation of the double-layer tubular structure of the present invention, and the mutual ratio of the thicknesses of the two layers, within such ranges as above reported, will vary according to the particular use, and hence to the mechanical characteristics required for the tubular structure.

For example, in haemodialysis apparatuses, lengths of tubular structures with different compositions will be preferably used, according to the particular treatment they shall undergo. In particular, the tubular structures to be used in the equipment area subject to the action of the peristaltic pump shall have a higher mechanical strength than, e.g., those lengths which are suitable for passive blood flow, and which may hence be softer.

The optimization of these parameters can be easily performed on by way of experiments by anyone skilled in this particular field.

However, the tubular structure useable in this field will be typically characterized by a hardness comprised within the range of from 55 to 90 degrees Shore A.

For exemplifying purposes, four double-layer tubular structures were prepared, which had different compositions:
A) a double-layer tubular structure having an inner diameter of 4.3 mm and an outer diameter of 6.8 mm, constituted by an inner layer of 1.75 mm of thickness of EVA at 15% of vinylic monomer, and by an outer layer of PVC of 0.75 mm of thickness, containing 35±2% of plastifier (DOP) and 6±1% of epoxydated soybean oil.
B) a double-layer tubular structure having an inner diameter of 4.3 mm and an outer diameter of 6.8 mm, constituted by an inner layer of 2.0 mm of thickness of EVA at 15% of vinylic monomer, and by an outer layer of PVC of 0.5 mm of thickness, containing 35±2% of plastifier (DOP) and 6±1% of epoxydated soybean oil.
C) a double-layer tubular structure having an inner diameter of 6.36 mm and an outer diameter of 9.54 mm, constituted by an inner layer of 2.12 mm of thickness of EVA at 20% of vinylic monomer, and by an outer layer of PVC of 1.06 mm of thickness, containing 38±2% of plastifier (DOP) and 8±1% of epoxydated soybean oil.
D) a double-layer tubular structure having an inner diameter of 6.36 mm and an outer diameter of 9.54 mm, constituted by an inner layer of 2.54 mm of thickness of EVA at 24% of vinylic monomer, and by an outer layer of PVC of 0.64 mm of thickness, containing 38±2% of plastifier (DOP) and 8±1% of epoxydated soybean oil.

The tubular structures were prepared with a co-extrusion equipment using co-extrusion heads with the proper cross-section surface area and diameters of the extrusion bores, separately charging the two extruders with the PVC and the EVA having the above-reported compositions. The extrusion of PVC is carried out within a temperature range of from 140 to 160°C, and the extrusion of EVA is carried out at a temperature comprised within the range of from 200 to 220°C. At the outlet of the co-extrusion head, maintained at the controlled temperature of 160°C, inside the tubular structure an air pressure slightly higher than atmospheric value is maintained. At 10 cm of distance from the co-extrusion head a 5 metre long water cooling bath is placed, through which the co-extruded tubular structure is made run, under a slight pull.

The so obtained (A) and (C); and (B) and (D) tubular structures were used for preparing two different haemodialysis apparatuses, wherein the (A) and (B) tubular structure are used for the passive blood flow, and the (C) and (D) tubular structures are used for that portion of the apparatus which is subject to the action of the peristaltic pump.

These apparatuses were tested, according to the test methodology described by Italian Pharmacopoeia, IX Ed., pages 498-503, by comparison with a conventional equipment wherein the tubular portion is wholly constituted by PVC (inner and outer diameter of the tubular element used for passive blood flow: 4.3 and 6.8 mm; and PVC composition corresponding to that of the outer layer of (A) and (B) tubular structures; inner and outer diameter of the tubular element subject to the action of the peristaltic pump: 6.36 and 9.54 mm; and PVC composition corresponding to that of the outer layer of (C) and (D) tubular structures), in order to evaluate the amount of phthalates in the eluate from the three different devices.

It was found that in both the apparatuses prepared by using the new tubular structures of the present invention, a drastic reduction occurs in the end amount of phthalates, relatively to the conventional equipment used for comparison purposes, with such reduction ranging from 70 to 90%.

The mechanical characteristics of the new tubular structures demonstrated to be very good, both as regards the mechanical strength of the tubular part submitted to the action of the peristaltic pump, and as regards the possibility of assemblage of the various elements by glueing and the perfect tightness of the so-made connections.

## Claims

1. Double-layer tubular structure of plastics material, characterized in that an outer layer of PVC containing an amount of plastifiers comprised within the range of from 25 to 40% is coupled with an inner layer of EVA containing from 6.5 to 40% of vinylic monomer, and that the thickness of the inner layer is comprised within the range of from 35 to 95% of the total thickness of the tubular structure.

2. Tubular structure according to claim 1, wherein the thickness of the inner layer is comprised within the range of from 70 to 85% of the total thickness of the tubular structure.

3. Tubular structure according to claim 1 or 2, wherein EVA contains from 10 to 25% of vinylic monomer.

4. Use of the tubular structure according to any of the preceding claims, for preparing apparatuses destined to bio-medical use.

5. Apparatus for haemodialysis, wherein the connections are constituted by tubular structures of plastics material according to any of the preceding claims from 1 to 3.

6. Method of preparation of a tubular structure according to any of the preceding claims from 1 to 3, wherein the two thermoplastic materials are separately heated to a temperature at least equal to their respective melting temperature, and are simultaneously co-extruded through a suitable co-extrusion head, and the so extruded tubular structure is finally cooled.

7. Method according to claim 6, wherein the cooling of the tubular structure is carried out by making said tubular structure run, under a slight pull, through a water cooling bath.

8. Method according to claim 7, wherein a slight pressure is maintained inside the tubular structure, along the length thereof running from the extrusion head to the cooling bath.

## Patentansprüche

1. Doppellagiger rohrförmiger Gegenstand aus Kunststoff, dadurch gekennzeichnet, daß eine Außenschicht aus PVC mit einem Gehalt an Weichmacher im Bereich von 25 bis 40% verbunden ist mit einer Innenschicht aus EVA mit einem Gehalt von 6.5 bis 40% an Vinylmonomeren und daß die Dicke der Innenschicht 35 bis 95% der Gesamtdicke des rohrförmigen Gegenstands beträgt.

2. Rohrförmiger Gegenstand nach Anspruch 1, bei dem die Dicke der Innenschicht 70 bis 85% der Gesamtdicke des rohrförmigen Gegenstands beträgt.

3. Rohrförmiger Gegenstand nach Anspruch 1 oder 2, bei dem EVA 10 bis 25% an Vinylmonomeren enthält.

4. Verwendung des rohrförmigen Gegenstands nach einem der vorhergehenden Ansprüche bei der Herstellung von für biomedizinischen Gebrauch bestimmten Apparaten.

5. Apparat für die Hämodialyse, bei dem die Verbindungen hergestellt sind durch rohrförmige Gegenstände aus Kunsttstoff nach einem der vorhergehenden Ansprüche 1 bis 3.

6. Verfahren zur Herstellung eines rohrförmigen Gegenstands nach einem der vorhergehenden Ansprüche 1 bis 3, bei dem die beiden thermoplastischen Stoffe getrennt auf eine Temperatur erhitzt werden, die wenigstens gleich ihrer jeweiligen Schmelztemperatur ist, und gleichzeitig durch einen geeigneten Koextrusionskopf koextrudiert werden, wobei der so extrudierte rohrförmige Gegenstand schließlich abgekühlt wird.

7. Verfahren nach Anspruch 6, bei dem das Abkühlen des rohrförmigen Gegenstands dadurch erfolgt, daß der rohrförmige Gegenstand mit leichtem Zug durch ein Wassserkühlbad hindurchgeführt wird.

8. Verfahren nach Anspruch 7, bei dem ein leichter Druck innerhalb des rohrförmigen Gegenstands auf der Länge hiervon aufrecht erhalten wird, die sich vom Extrusionskopf zum Kühlbad erstreckt.

## Revendications

1. Structure tubulaire en matériau plastique, caractérisée en ce qu'une couche extérieure en PVC contenant une quantité de plastifiants comprise entre 25 et 40 %, est mariée à une couche intérieure constituée d'EVA contenant de 6,5 à 40 % de monomère vinylique, et en ce que l'épaisseur de la couche intérieure est comprise entre 35 à 95 % de l'épaisseur totale de la structure tubulaire.

2. Structure tubulaire selon la revendication 1, caractérisée en ce que l'épaisseur de la couche intérieure est comprise entre 70 à 85 % de l'épaisseur totale de la structure tubulaire.

3. Structure tubulaire selon la revendication 1 ou 2, caractérisée en ce que l'EVA contient entre 10 et 25 % de monomère vinylique.

4. Utilisation de la structure tubulaire selon l'une des revendications précédentes, pour la préparation d'appareils destinés à l'usage bio-médical.

5. Appareil d'hémodialyse, dans lequel les connections sont constituées de structures tubulaires en matériau plastique selon l'une des revendications précédentes 1 à 3.

6. Procédé de préparation d'une structure tubulaire selon l'une des revendications précédentes 1 à 3, dans lequel on chauffe séparément les deux matériaux thermoplastiques à une température au moins égale à leur température de fusion respective, on les co-extrudant simultanément à travers une tête de co-extrusion appropriée, puis on refroidit finalement la structure tubulaire ainsi extrudée.

7. Procédé selon la revendication 6, dans lequel le refroidissement de la structure tubulaire est réalisé en la faisant passer sous une poussée légère à travers un bain de refroidissement à eau.

8. Procédé selon la revendication 7, dans lequel on maintient une très faible surpression d'air à l'intérieur de la structure tubulaire, sur toute la longueur s'étendant depuis la tête d'extrusion jusqu'au bain de refroidissement à eau.
